# EUROPEAN PATENT SPECIFICATION

(11) **EP 0 639 385 B1**
(45) Date of publication and mention of the grant of the patent: **21.06.2000**
(21) Application number: 94112676.5
(22) Date of filing: 13.08.1994
(51) Int. Cl.: A61M 5/00, A61M 39/00

(54) **Container for stopper for medical purpose**
Behälter für medizinische Kappe
Etui pour capuchon à usage médical

(30) Priority: 20.08.1993 JP 22788793
(43) Date of publication of application: 22.02.1995
(73) Proprietor: KANAE COMPANY LTD, Osaka-shi, Osaka (JP)
(72) Inventor: Ishiodori, Tetsushi, c/o Kanae Company Ltd., Osaka-shi, Osaka (JP); Maeda, Kazumasa, c/o Kanae Company Ltd., Osaka-shi, Osaka (JP); Takaishi, Kazuki, c/o Kanae Company Ltd., Osaka-shi, Osaka (JP)
(74) Representative: Gille Hrabal Struck Neidlein Prop Roos

(56) References cited:
- DE-B- 1 187 765
- GB-A- 1 165 258
- US-A- 4 444 310
- US-A- 4 938 745

## Description

### BACKGROUND OF THE INVENTION

### 1. Field of the Invention

A tubing for supplying liquid to and taking out liquid from artificial organs implanted in a human body (the tubing is simply called a "tube" hereunder) is extended from the body occasionally through skin. In the medical treatment as supplying or taking liquid into or from a subject's body, the tube is connected at the utmost end with a liquid-containing bag,a pump or the like. When the treatment finishes, the utmost end of the tube becomes in the state of being merely open. The open end of the tube is possibly invaded with various bacteria, etc., so that the utmost end is to be always tightly closed by a sanitary stopper means after the medical treatment finishes.

The present invention provides a cheap and sanitary packaging container for the stopper means.

### 2. Description of the Related Art

The stoppers are small in size and preserved as spares. Upon use of the stopper, its package is broken and the stopper is then used properly and thereafter disposed or burnt. Since the stoppers themselves are relatively cheap, the packages for the stoppers are also to be cheap and functionally to be severely free of bacteria or sterile and have easiness in use.

The stoppers which are used in the above way are each preserved and packaged conventionally by use of a bag which is designed to have a tightly sealing-up efficiency and contains and preserves the stopper.

After the medical treatment finishes, an operator opens the bag and takes out the stopper therefrom, and puts the stopper on the open utmost end of the tube with his or her hand holding the stopper, so as to fit the stopper to the utmost end of the tube.

The conventional method has such defect in large possiblity that the stopper and tube are contaminated with various bacteria existing on the operator's hand when taking out the stopper from the bag and fitting the stopper to the tube with the hand holding the stopper. Hence, a more rational packaging means for the stopper has been desired.

US-A-4444310 discloses a container corresponding to the preamble of claim 1.

### SUMMARY OF THE INVENTION

The present invention, as characterized in claim 1, provides a packaging container for the stopper to be used in the foregoing way, the packaging container being cheap and having a novel structure as readily usable and sanitary in use.

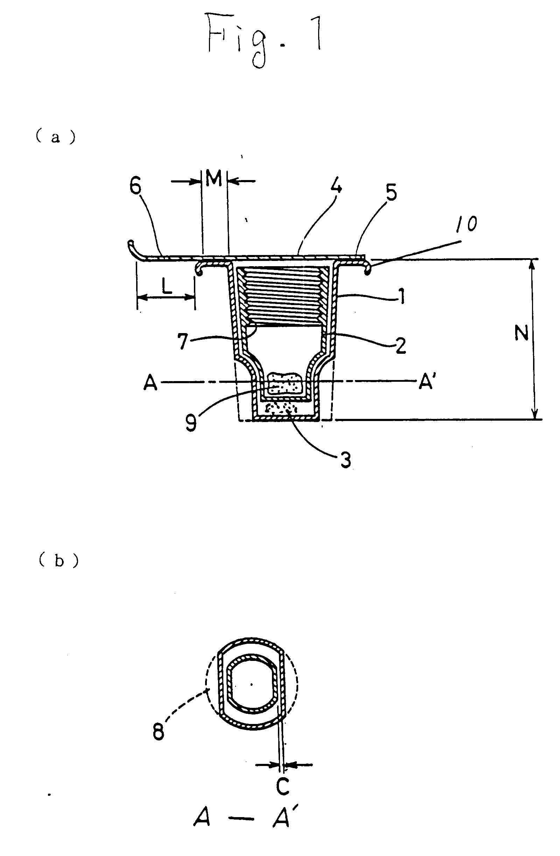

A container according the first invention comprises, as shown in Fig. 1, a tubular container body which has a flat chamfered part on the opposite surfaces near the lower end of the container, the upper edge of the container extending outwardly and perpendicularly to the axis of the container, the end part of the upper edge being turned as radiused, and a detachable lid 4 being connected to the upper edge of the container by a proper welding or adhesive means.

The detachable lid 4 has an ear part 6 which is to be pulled to readily open the lid of the container for use of the stopper. The radiused part of the end of the upper edge of the container is proper for enabling the detachable lid to be adhered to the upper edge as having no gaps on the whole surface.

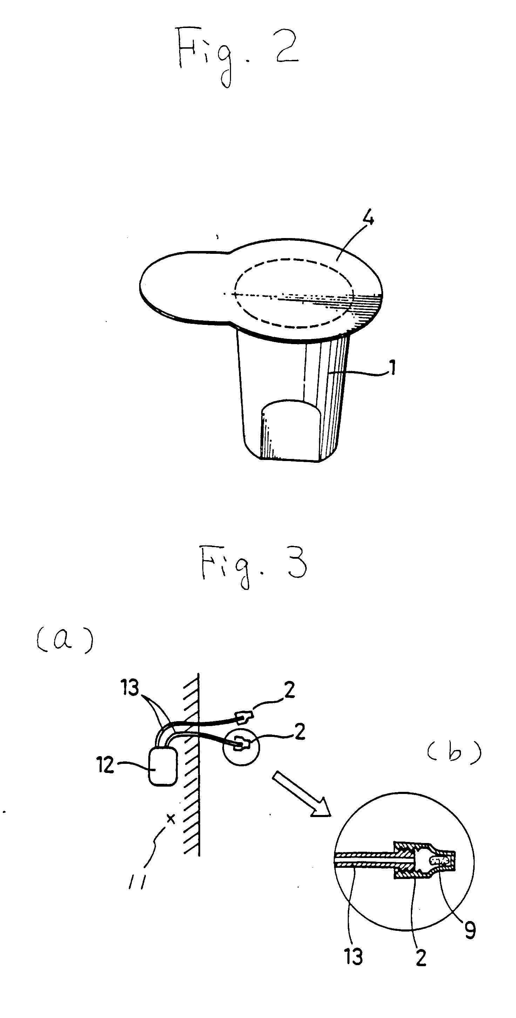

After removing the lid, the stopper 2 in the state of being packaged in the container 1 is put on the utmost end of the tube (13 in Fig. 3). The container 1 is turned to turn the stopper 2 simultaneously so as to cause the stopper to be tightly screwed on the tube end. As shown in Fig. 1b, both the container and the stopper are provided with the chamfered part and are fit to each other, so that an operator turns the container with holding its outside to fit the stopper onto the tube end sanitarily without idling of the stopper and necessity of operator's touching the stopper.

Gap C between the container and the stopper is preferably in the range of - 0.1 to 0.7mm which dimension is just proper for the purpose of the invention and does not cause backlash in operation and transportation and idling of the stopper upon mounting to the tube end. Also, the dimension allows that after the stopper is mounted to the tube, the container can be removed smoothly. The dimension - 0.1mm of the gap C means that the outer surface dimension of the chamfered part of the stopper 2 at its lower end is set to be slightly larger (by about 0.1mm) than the inner surface dimension of the corresponding chamfered part of the container 1. According to the setting, the stopper has no backlash and is not unexpectedly thrown out by an impact upon opening the lid, and the container when removed after mouting the stopper to the tube end is not subjected to high resistance. In case that the dimension C is set to be smaller than - 0.1mm, the container when removed after mounting the stopper to the tube is subjected to high resistance.

Examples of the materials of the container 1 include, in consideration of being readily processed for shaping and of impermeability to various bacteria, air, etc., thermoplastic polymeric materials, thermosetting polymeric materials, polyethylene, polypropylene, polyvinyl chloride, polystyrene, ABS resin, PBT resin, PET resin and other engineering plastics. Ceramics may be usable in consideration of strength.

Other than the above, thin plates made of glass or aluminium may be pressed into the above shapes and preferably used in respect of improving a barrier efficiency against the outside.

Furthermore, the container obtained by pressing the aluminium thin plate into the above shape may be coated at its inner surface and/or inside and outside with with a macromolecular film for providing a preferable function. In detail, a three-layered material comprising layers of polyethylene terephthalate (PET) layered on the inner and outer surfaces of the container may be a most preferable material in respect of bonding propeties with the lid and a barrier efficiency against ambient air.

In either cases using the detachable lid or a modified feature, a knobbed lid in disc-like shape, the container may be provided on the inside of the upper part with a plurality of protuberances 24 in order to prevent the stopper from unexpectedly falling or sliding down from the container upon opening the lid or in the mounting process to the tube. Inner diameter of a circle formed by the protuberances 24 is the same in dimension as of the outer diameter of the opening of the upper edge of container or slightly smaller than the latter (by about 0.1mm). Hence, after mounting the stopper to the tube, the container can be readily removed by pulling the container with lightly depressing outside parts of the container not corresponding to the protuberances or without depressing in the manner.

The material for the lid 4 is required to have strength enough not to be torn in pieces when the lid is pulled for peeling, in addition to the requirements for the materials of the container. In detail, a layer-built sheet comprising PET-aluminium - PET- a seal coating is preferable.

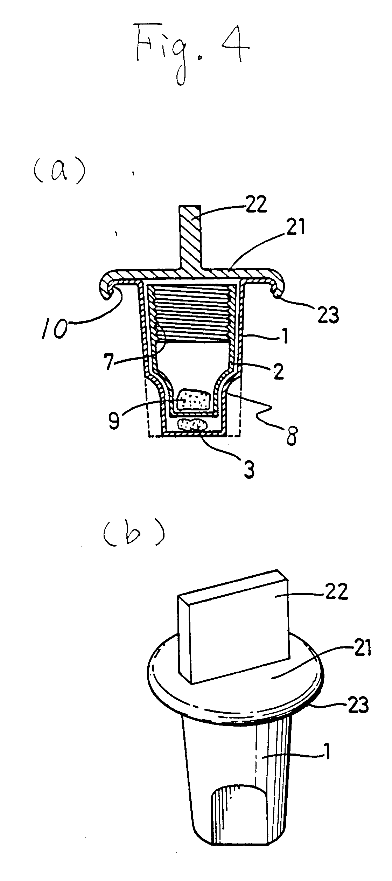

Next, a container according to the second invention comprises, as shown in Fig. 4a, a tubular container body which has a flat chamfe red part on the opposite surfaces near the lower end of the container, the upper edge of the container extending outwardly and perpendicularly to the axis of the container, the end part of the upper edge being turned as radiused 10, and a disc-like shaped lid 21 having a knob in a plate and projection-like shape being fit on the upper edge of the container, as shown in Figs. 4a and 4b.

Inner diameter of the radiused part 23 of the lid is set to be smaller than that of the radiused part 10 of the end of the extended edge of the upper opening of the container, so that the lid and the upper edge of container can be fit to each other air-tightlyand detachably through elasticity and adherence of the lid.

Materials and structures for the container 1 may be the same as in the case using the detachable lid.

Materials for the lid 21 and 22 are required to have some elasticity and adherence in consideration of that the edge 23 of the lid is to be fit air-tightly to the end 10 of the extended edge of the upper opening of the container in a manner of being expanded somewhat. Examples of the materials are polyethylene, soft polyvinyl chloride resin, acryl · butadiene (AB) copolymer resin, etc. Synthetic polymeric materials having flexibility and some elasticity may be usable. Furthermore, a film made of laminated aluminium may be preferably adhered or inserted as a packing on the inner side of the lid 21 and between the lid 21 and the extended edge part of the container 1, so as to improve the barrier effieicncy for the container.

### BRIEF DESCRIPTION OF THE DRAWINGS

Fig. 1a is a longitudinally sectional view showing a container according to the present invention containing a stopper therein, Fig. 1b a longitudinally sectional view taken from the line A-A' in Fig. 1a.
Fig. 2 is a perspective view of the container on which a lid is connected.
Fig. 3a shows a tube extended from an apparatus implanted in a human body to the outside and a stopper fit to the utmost end of the tube. Fig. 3b is an enlarged view of the part in Fig. 3a as circled, showing the stopper fit onto the tube.
Fig. 4a is a longitudinally sectional view of a container using a disc-like shaped lid having a knob, Fig. 4b a perspective view of the same.

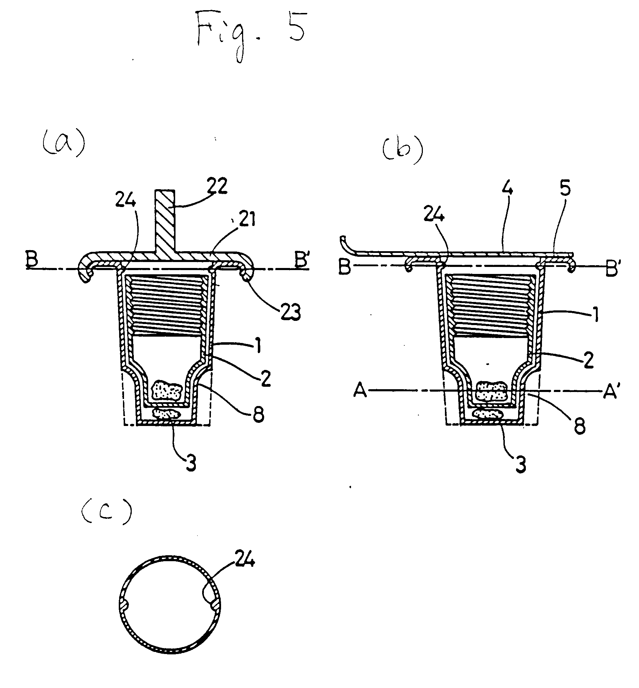

Fig. 5a is a side elevational view of a container having the knobbed lid and provided with protuberances inside the container, Fig. 5b a side elevational view of a container having the detachable lid and provided with protuberances inside the container, and Fig. 5c a sectional view of the container taken in the line B-B'.

### DETAILED DESCRIPTION OF THE PREFERRED EMBODIMENTS

### Example 1

Next, an example of the present invention will be detailed with referring to the drawings.

Fig.2 is a perspective view showing a first example of the container according to the present invention, Fig. 1a a longitudinally sectional view of the same and Fig. 1b a cross-sectional view taken in the line A-A' in Fig. 1a.

The packaged object 2 (Stopper) is inserted into a sterile or bacteria-free packaging container 1. A detachable lid 4 is adhered on the upper edge of the container in such manner that the lid 4 can be readily peeled off from the container.

The stopper 2 is mounted to a tube extended from any object in a human body generally by use of thread means so as to be prevented from falling. The stopper is provided with thread 7 near the edge of the upper opening, and with a chamfered part 8 below the thread part 7 for enabling the thread part 7 to be readily turned.

The sterile container 1 is hard and in a shape similar to the stopper as having chamfered parts on the lower opposite surfaces of the tubular container body to contain the stopper 2 properly. Gap between the stopper 1 and the container 2 is designed to be about 0.5mm as a whole.

When the stopper is to be mounted onto any tube as foregoing after peeling off the detachable lid 4, provision of the chamfered parts enables the stopper in the state of being contained in the container to be fit onto the utmost open end of the tube without necessity of taking out the stopper from the container and touching the stopper.

### Example 2

Figs. 4a and 4b show a second example of the container according to the invention. The container is identical to that referred to in the Example 1.

In this example, a lid 21 is disc-like shaped and has a plate-like shaped knob 22, and the edge end 23 is radiused downwardly, so that the lid 21 can be fit to the container detachably and air-tightly through the end of the extended edge of the upper opening of the container. The plate-like knob 22 is a surplus for enabling the lid to be readily detached from the container body. When the knob 22 is relatively larger in size, the lid is more readily opened. And the knob 22 when designed to be larger in size enables the lid to be readily detached from the container even when fitting efficiency and adherence between the container body and the lid are made higher to improve airtight condition.

The invention is constructed as explained above and has the following effects. In detail, the containers structured as set forth in the claims enable the medical purpose stopper to be fit, in a completely bacteria-free or sterile condition, onto the utmost end of the tube extended from the subject body, thereby enabling the medical treatment in a cheap, simple and sanitary condition.

## Claims

1. Container (1) for packaging a medical purpose stopper (2), said container being provided with a detachable adhered lid (4) having an airtight structure and including a tubular and hard container body,
**characterized in that**
the container (1) body has a flat chamfered part (8) on opposite surfaces near the lower end of the container.

2. Container as claimed in claim 1, characterized in that the tubular and hard container body (1) having a flat chamfered part on opposite surfaces near the lower end of the container has a plurality of protuberances (24) provided near the upper part on the inner side of the container (1).

3. Container as claimed in claim 1 or 2, characterized in that the detachable lid (4) mounted to the container (1) is provided with a plate-like shaped projecting knob (22) and has an airtight and freely detachable structure.

## Patentansprüche

1. Verpackung (1) für einen medizinischen Stöpsel (2), die mit einem abreißbaren Verschluss (4) mit luftdichter Struktur ausgestattet ist und einen schlauchförmigen harten Verpackungskörper besitzt,
**dadurch gekennzeichnet, dass**
die Verpackung (1) an gegenüberliegenden Seiten am unteren Ende der Verpackung einen flachen abgeschrägten Teil (8) aufweist.

2. Verpackung nach Anspruch 1,
**dadurch gekennzeichnet, dass**
der schlauchförmige harte Körper der Verpackung (1), der einen flachen abgeschrägten Teil an den gegenüberliegenden Seiten am unteren Ende der Verpackung besitzt, eine Vielzahl von Vorsprüngen (24) aufweist, die in der Nähe des oberen Teils an der Innenseite der Verpackung (1) angeordnet sind.

3. Verpackung nach einem der Ansprüche 1 oder 2,
**dadurch gekennzeichnet, dass**
der an der Verpackung (1) befestigte abreißbare Verschluss (4) mit einem plattenförmigen vorstehenden Knopf (22) ausgerüstet ist und eine luftdichte frei abreißbare Struktur hat.

## Revendications

1. Etui (1) destiné à encapsuler un capuchon à utilisation médicale (2), ledit étui étant muni d'un couvercle détachable adhérant (4) de structure étanche à l'air et comprenant un corps d'étui tubulaire dur, caractérisé en ce que le corps d'étui (1) possède une partie biseautée plate (8) disposée sur des surfaces opposées et située à proximité de l'extrémité inférieure de l'étui.

2. Etui selon la revendication 1, caractérisé en ce que le corps d'étui tubulaire dur (1) possédant une partie biseautée plate disposée sur des surfaces opposées et située à proximité de l'extrémité inférieure de l'étui a une pluralité de protubérances (24) placées à proximité de la partie supérieure sur le côté intérieur de l'étui (1).

3. Etui selon la revendication 1 ou 2, caractérisé en ce que le couvercle détachable (4) monté sur l'étui (1) est pourvu d'un bouton (22) faisant saillie et en forme de plaque et possède une structure étanche à l'air et qui peut se détacher librement.
